# EUROPEAN PATENT SPECIFICATION

(11) **EP 1 473 001 B1**
(45) Date of publication and mention of the grant of the patent: **15.11.2006**
(21) Application number: 04252458.7
(22) Date of filing: 28.04.2004
(51) Int. Cl.: A61F 5/44

(54) **Medical waste collection device**
Vorrichtung zur Sammlung von medizinischen Abfällen
Dispositif de récupération de déchets médicaux

(30) Priority: 29.04.2003 GB 0309800
(43) Date of publication of application: 03.11.2004
(60) Divisional application: 06018552.7
(73) Proprietor: Mentor Medical Limited, Lancing, West Sussex BN15 8TJ (GB)
(72) Inventor: French, Nigel, Lancing, West Sussex BN15 8TJ (GB)
(74) Representative: Evans, Marc Nigel

(56) References cited:
- US-A- 4 306 705
- US-A- 4 844 415

## Description

The present invention relates to a waste collection device, particularly to a waste collection bag device for collecting fluids, such as urine, discharged from a human body, such as described in US-A- 4306705.

Waste collection bags of the kind used in the medical field can by virtue of their construction be drained by either tearing or cutting the bag thereby at the same time preventing reuse of the bag.

It is an aim of the present invention to provide an improved construction for a waste collection bag device that is intended to be disposed of after a single use.

The present invention provides a medical waste collection device, including a collection bag having an inlet and a separate outlet, and a closed tap at the outlet of the bag to block, in use, the flow of waste fluid through the outlet, the closed tap being controllably openable to allow, in use, fluid collected in the bag to be controllably drained from the bag, but being less easily reclosable to thereby deter reuse of the tap and bag.

The present invention also provides the use of such a waste collection device for collecting fluids discharged from a human body.

An embodiment of the present invention is described hereunder, by way of example only, with reference to the accompanying drawings, in which:-
Figure 1 shows a waste collection device according to an embodiment of the present invention with the tap in a closed position; and
Figure 2 shows the waste collection device of Figure 1 with the tap in the open position.

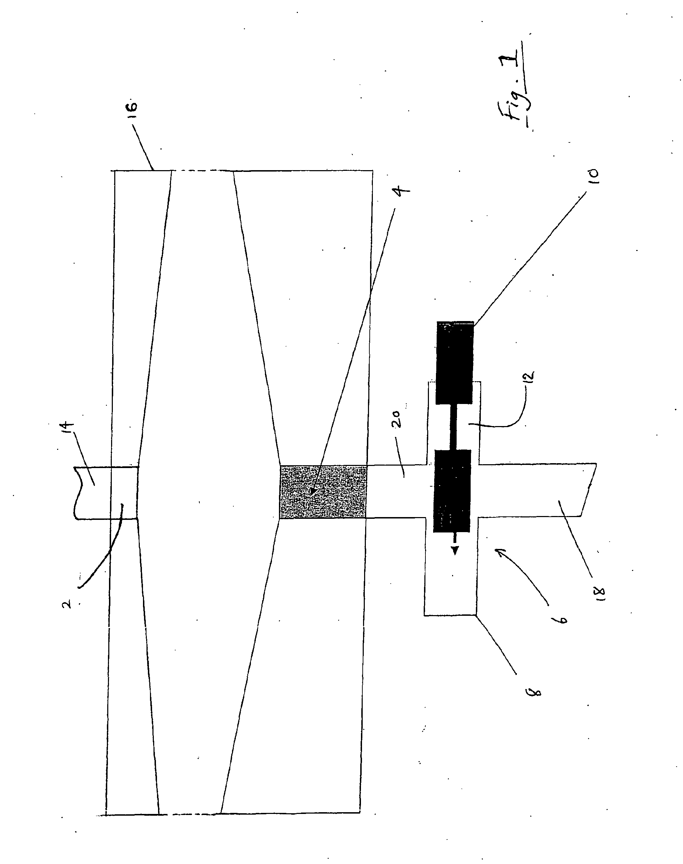

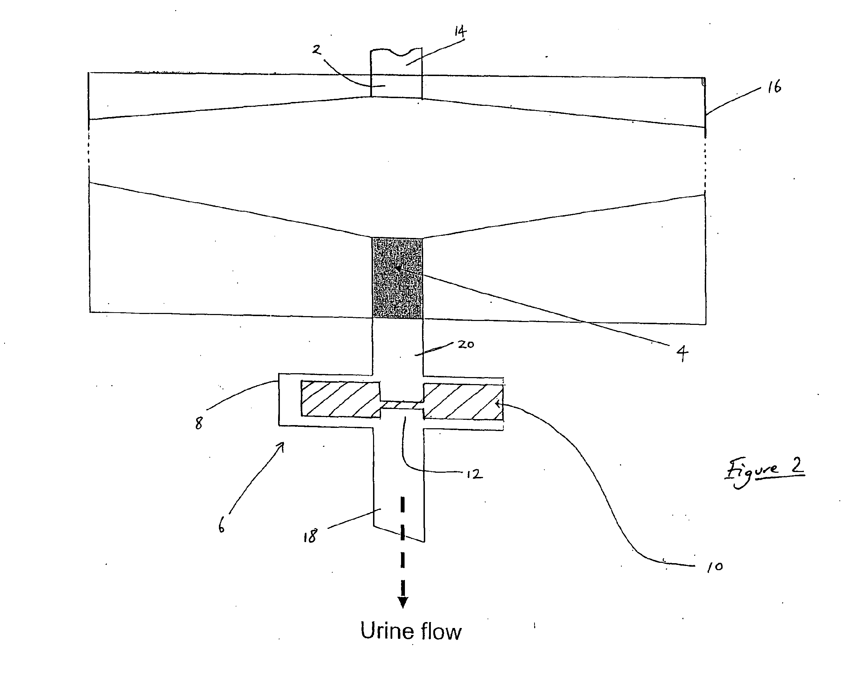

With reference to Figures 1 and 2, a waste collection device according to an embodiment of the present invention includes a collection bag 16 having an inlet at the top end and an outlet 4 at the bottom end. into the inlet 2 is permanently welded inlet hosing or tubing 14 for leading the waste fluid from the human body into the collection bag. Into the outlet 4 is permanently welded a tap 6. The tap includes a housing 8 which defines an outlet 18 and an inlet 20 which is connected to the interior of the collection bag via the bag outlet 4. In the housing is fitted a sliding actuator 10. The sliding actuator 10 includes a section of reduced diameter 12 which allows the flow of water from the inlet to the outlet when (at least partially) aligned with the inlet and outlet of the housing. The sliding actuator 10 is fitted in the housing 8 such that the actuator blocks the flow of fluid from the inlet to the outlet other than when the section of reduced diameter 12 is (at least partially) aligned with the inlet and outlet of the housing.

In use, the waste collection device is supplied with the tap in a closed position as shown in Figure 1. The inlet hosing or tubing is connected to the patient for directing waste fluid into the collection bag. The close fit of the actuator in the housing such that there is sufficient frictional force between the two components to prevent the tap being accidentally dislodged into the open position.

When the collection bag is full or all the fluid to be collected has been collected, the tap is opened by pushing the actuator in the direction shown by the arrow in Figure 1 via the portion of the actuator protruding from the housing 8. As shown in Figure 2, the tap is designed such that when the actuator has been pushed into the housing as far as it can be easily done so by hand, i.e. when the end of the actuator 10 is substantially aligned with the opening of the housing 8, the whole of the section of the actuator of reduced diameter 12 is substantially aligned with the outlet and inlet of the housing.

The housing 8 is closed at the other end such that the actuator 10 cannot be pushed in the opposite direction back into a closed position. The closed end of the housing is positioned such that it abuts with the respective end of the actuator when the tap is in the "open" position, thereby serving to also further prevent any further undesirable insertion of the actuator to a position where the flow of fluid is more restricted or completely blocked.

With this tap construction, it is very difficult for a user to inadvertently reuse the tap/bag/inlet tubing assembly, which reuse would generally be undesirable from the point of view of avoiding accidental contamination. With this waste collection device, the bag can be controllably drained by hand whilst at the same time effectively deterring reuse of the tap/bag/inlet tubing assembly.

Furthermore, the tap is of a simple construction which can be produced and incorporated into a collection bag at minimal cost whilst providing the above-mentioned advantages.

In one alternative embodiment, the tap is secured into the bag by mechanical means rather than welding. The tap could be a push fit into a tube previously welded into the bag.

In another alternative embodiment, the longitudinal actuator could be a solid rod provided with a transverse throughhole instead of a section of reduced diameter.

The applicant draws attention to the fact that the present invention may include any feature or combination of features disclosed herein either implicitly or explicitly or any generalisation thereof, without limitation to the scope of any definitions set out above. In view of the foregoing description it will be evident to a person skilled in the art that various modifications may be made within the scope of the invention, as defined by the appended claims.

## Claims

1. A medical waste collection device, including a collection bag (16) having an inlet (2) and a separate outlet (4), and a closed tap (6) at the outlet of the bag to block, in use, the flow of waste fluid through the outlet, **characterised in that** the closed tap is controllably openable to allow, in use, fluid collected in the bag to be controllably drained from the bag, but is less easily reclosable to thereby deter reuse of the tap and bag.

2. A medical waste collection device according to claim 1, wherein the tap is not reclosable by hand to thereby further deter reuse of the tap and bag.

3. A medical waste collection device according to claim 1, wherein the closed tap includes a housing (8) and a longitudinal insert (10) fitted in said housing in a first position in which the flow of fluid through the tap is blocked and a first longitudinal end of the insert protrudes from the housing; wherein the insert can be pushed longitudinally via the protruding first end thereof to a second position in which fluid can flow through the tap, and wherein the housing is structured so as to prevent access by hand to the other longitudinal end of the insert and thereby deter push operation of the insert in the opposite direction back to a closed position.

4. A medical waste collection device according to claim 3, wherein the portion of the housing receiving said other longitudinal end of the insert is closed so as to prevent access by hand to said other longitudinal end of the insert.

5. A medical waste collection device according to claim 3 or claim 4, wherein when the insert is fully pushed into the housing, the tap is in an open position.

6. The use of a waste collection device according to any preceding claim for collecting fluids discharged from a human body.

## Patentansprüche

1. Vorrichtung zum Sammeln medizinischer Ausscheidungsprodukte, aufweisend einen Sammelbeutel (16), der einen Einlaß (2) und einen separaten Auslaß (4) hat, sowie einen geschlossenen Abgriff (6) am Auslaß des Beutels, um im Gebrauch den Strom an fluidartigen Ausscheidungsprodukten durch den Auslaß zu blockieren, **dadurch gekennzeichnet, daß** der geschlossene Abgriff kontrollierbar zu öffnen ist, um im Gebrauch zu erlauben, daß in dem Beutel gesammeltes Fluid kontrolliert aus dem Beutel ablassbar ist, jedoch weniger leicht wieder zu schließen ist, um dabei die Wiederverwendung des Abgriffs und des Beutels zu behindern.

2. Vorrichtung zum Sammeln medizinischer Ausscheidungsprodukte nach Anspruch 1, bei welcher der Abgriff von Hand nicht wieder zu schließen ist, um dabei weiter eine Wiederverwendung des Abgriffs und Beutels zu behindern.

3. Vorrichtung zum Sammeln medizinischer Ausscheidungsprodukte nach Anspruch 1, bei welcher der geschlossene Abgriff ein Gehäuse (8) und einen länglichen Einsatz (10) aufweist, der in das Gehäuse gepaßt ist in einer ersten Stellung, in welcher der Strom an Fluid durch den Abgriff blockiert und ein erstes längliches Ende des Einsatzes von dem Gehäuse vorsteht; wobei der Einsatz über sein vorstehendes, erstes Ende in Längsrichtung in eine zweite Stellung verschiebbar ist, in welcher Fluid durch den Abgriff strömen kann und wobei das Gehäuse derart aufgebaut ist, daß es einen Zugriff von Hand an dem anderen länglichen Ende des Einsatzes verhindert und dabei einen Schiebevorgang des Einsatzes in die entgegengesetzte Richtung, zurück in die geschlossene Stellung, behindert.

4. Vorrichtung zum Sammeln medizinischer Ausscheidungsprodukte nach Anspruch 3, bei welcher der Abschnitt des Gehäuses, der das längliche Ende dem Einsatzes aufnimmt, geschlossen ist, um einen Zugriff von Hand an dem anderen länglichen Ende des Einsatzes zu verhindern.

5. Vorrichtung zum Sammeln medizinischer Auoscheidungsprodukte nach Anspruch 3 oder Anspruch 4, bei welcher der Abgriff in einer offenen Stellung ist, wenn der Einsatz vollständig in das Gehäuse geschoben ist.

6. Verwendung einer Vorrichtung zum Sammeln von Ausscheidungsprodukten nach einem der vorstehenden Ansprüche zum Sammeln von Fluiden, die von einem menschlichen körper abgegeben wurden.

## Revendications

1. Dispositif de recueil de déchets médicaux incluant un sac de recueil (16) ayant une entrée (2) et une sortie séparée (4) et un robinet fermé (6) à la sortie du sac pour bloquer, en cours d'utilisation, l'écoulement de fluide rejeté par la sortie, **caractérisé en ce que** le robinet fermé est ouvrable de façon commandable pour permettre, en utilisation, au fluide recueilli dans le sac d'être extrait de façon contrôlable du sac mais est moins facilement refermable pour éviter ainsi la réutilisation du robinet et du sac.

2. Dispositif de recueil de décrets médicaux selon la revendication 1, dans lequel le robinet n'est pas refermable à la main pour éviter ainsi une utilisation ultérieure du robinet et du sac,

3. Dispositif de recueil de déchets médicaux selon la revendication 1, dans lequel le robinet fermé comprend un boîtier (8) et un insert longitudinal (10) disposé dans le boîtier à une première position dans laquelle l'écoulement du fluide à travers le robinet est bloqué, et une première extrémité longitudinale de l'insert fait saillie à partir du boîtier, dans lequel l'insert peut être poussé longitudinalement par sa première extrémité en saillie vers une seconde position dans laquelle le fluide peut s'écouler par le robinet, et dans lequel le boîtier est agencé pour éviter un accès manuel vers l'autre extrémité longitudinale de l'insert et éviter ainsi une opération de poussée de l'insert en direction opposée en retour vers une position fermée.

4. Dispositif de recueil de déchets médicaux selon la revendication 3, dans lequel la partie du boîtier recevant l'autre extrémité longitudinale de l'insert est fermée de façon à éviter un accès à la main vers l'autre extrémité longitudinale de l'insert.

5. Dispositif de recueil de déchets médicaux selon la revendication 3 ou 4, dans lequel, quand l'insert est complètement poussé dans le boîtier, le robinet est en position ouverte.

6. Utilisation d'un dispositif de recueil de déchets selon l'une quelconque des revendications précédentes, pour recueillir des fluides sortant d'un corps humain.
